# EUROPÄISCHE PATENTSCHRIFT

(11) **EP 3 768 199 B1**
(45) Veröffentlichungstag und Bekanntmachung des Hinweises auf die Patenterteilung: **28.12.2022**
(21) Anmeldenummer: 19712163.5
(22) Anmeldetag: 15.03.2019
(51) Int. Cl.: A61F 5/01

(54) **POSITIONIEREINRICHTUNG UND SYSTEM AUS POSITIONIEREINRICHTUNG UND GRUND-KÖRPER EINER ORTHOPÄDIETECHNISCHEN EINRICHTUNG**
POSITIONING DEVICE AND SYSTEM FORMED OF A POSITIONING DEVICE AND BASE BODY OF AN ORTHOPAEDIC DEVICE
DISPOSITIF DE POSITIONNEMENT ET SYSTÈME COMPRENANT UN DISPOSITIF DE POSITIONNEMENT ET UN CORPS DE BASE D'UN DISPOSITIF ORTHOPÉDIQUE

(30) Priorität: 20.03.2018 DE 102018106574
(43) Veröffentlichungstag der Anmeldung: 27.01.2021
(73) Patentinhaber: Otto Bock Healthcare Products GmbH, 1110 Wien (AT)
(72) Erfinder: OVERDEVEST, Etienne, 37073 Göttingen (DE)
(74) Vertreter: Gramm, Lins & Partner Patent- und Rechtsanwälte PartGmbB
(86) Internationale Anmeldenummer: PCT/EP2019/056580
(87) Internationale Veröffentlichungsnummer: WO 2019/179893

(56) Entgegenhaltungen:
- WO-A1-2008/061300
- DE-T2- 69 434 390
- DE-U1-202007 005 891

## Beschreibung

Die Erfindung betrifft eine Positioniereinrichtung zum Anordnen und Ausrichten mehrerer Befestigungseinrichtungen auf einer Basislage eines Grundkörpers einer orthopädietechnischen Einrichtung sowie ein System aus Positioniereinrichtung und einem Grundkörper einer orthopädietechnischen Einrichtung, wie in den Ansprüchen definiert. Als orthopädietechnische Einrichtung werden insbesondere Orthesen oder Prothesen angesehen, grundsätzlich ist es auch möglich, die Positioniereinrichtung in Verbindung mit anderen orthopädietechnischen Einrichtungen, beispielsweise Exoskelette einzusetzen.

Orthesen- oder Prothesenkomponenten zur Aufnahme von oder zur Befestigung an einem Körperteil sind insbesondere Prothesenschäfte, in die ein Stumpf einer Gliedmaße eingeführt wird, oder Orthesenschalen oder Spangen, die an den Körper angelegt und daran befestigt werden, um gelenkübergreifend mit einer zweiten Orthesenkomponente über eine Gelenkeinrichtung verbunden zu werden. Prothesenschäfte werden häufig aus faserverstärkten Kunststoffen hergestellt, die auf Träger aufgelegt, mit Harz getränkt und anschließend ausgehärtet werden. Die Träger können als Standardvorlagen ausgebildet oder auf Basis eines Abgusses des jeweiligen Stumpfes erstellt werden. An einem distalen Ende des Prothesenschaftes wird eine Ankerplatte zur Festlegung eines Pyramidenadapters oder einer Adapteraufnahme befestigt oder eingegossen, so dass der Prothesenschaft mit einer distalen Gelenkeinrichtung und distalen Prothesenkomponenten verbunden werden kann.

Orthesenkomponenten zur Aufnahme von Körperteilen oder zur Befestigung an Körperteilen können aus Kunststoffkomponenten hergestellt sein. Ebenfalls können diese Komponenten, die als Schalen oder Spangen oder dergleichen ausgebildet sein können, aus faserverstärkten Kunststoffmaterialien hergestellt sein. Häufig sind die Orthesenkomponenten über Befestigungseinrichtungen wie Gurte oder Schnallen an dem Körperteil festlegbar. Über die Befestigungseinrichtungen wird die Gliedmaße umschlossen, gegebenenfalls werden die Orthesenkomponenten elastisch verformt. Dementsprechend können die Orthesenkomponenten in einem begrenzten Maße elastisch verformbar sein.

Zur Herstellung von Orthesen mit Orthesenkomponenten zur Anlage und Aufnahme von Körperteilen oder Gliedmaßen werden bei individuell angefertigten Orthesen und Gelenkeinrichtungen zusammen mit den Orthesenkomponenten auf einem Modell der Gliedmaße befestigt und einlaminiert. Alternativ werden Befestigungselemente für Gelenkeinrichtungen über Fixiereinrichtungen, sogenannte Dummies oder Platzhalter zueinander orientiert gehalten, die bei der Aushärtung und Fertigstellung der Orthesenkomponenten an Ort und Stelle verbleiben müssen.

Die DE 20 2007 005 891 U1 betrifft eine Frakturschiene mit einer Schiene und einer Positioniereinrichtung, die zwei über die Positioniereinrichtung schwenkbar zueinander gelagerte Elemente aufweist. Der Schwenkbereich der beiden Elemente zueinander wird durch die Positioniereinrichtung begrenzt.

Die DE 694 34 390 T2 betrifft eine Orthese mit zwei einander gegenüberliegenden Schienen, die jeweils ein Gelenk aufweisen. Die Gelenke verbinden Schienenabschnitte, die Gurte aufnehmen, damit diese Schienenabschnitte an einem Oberschenkel und einem Unterschenkeln festgelegt werden können. Ein Polster ist zwischen den Schienen und der Gliedmaße angeordnet. Der Schwenkbereich der Gelenke ist einstellbar.

Aufgabe der vorliegenden Erfindung ist es, eine Positioniereinrichtung zum Anordnen und Ausrichten mehrerer Befestigungseinrichtungen auf einer Basislage einer orthopädietechnischen Einrichtung sowie ein System aus einer Positioniereinrichtung und einem Grundkörper bereitzustellen, mit denen Befestigungseinrichtungen für zusätzliche Komponenten im Rahmen zulässiger Parameter zueinander positioniert werden können, um eine Individualisierung der orthopädietechnischen Einrichtung auf eine einfache Art und Weise zu ermöglichen.

Erfindungsgemäß wird diese Aufgabe durch eine Positioniereinrichtung mit den Merkmalen des Hauptanspruches und ein System mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die erfindungsgemäße Positioniereinrichtung zum Anordnen und Ausrichten mehrerer Befestigungseinrichtungen auf einer Basislage eines Grundkörpers einer orthopädietechnischen Einrichtung, mit einem ersten Halter, der zumindest eine Aufnahmeeinrichtung für ein Befestigungselement aufweist, und einem zweiten Halter, der zumindest eine Aufnahmeeinrichtung für ein Befestigungselement aufweist, sieht vor, dass der erste Halter und der zweite Halter ausgehend von einer Ausgangsstellung um einen eingeschränkten Winkelbereich um eine Verschwenkachse verschwenkbar aneinander gelagert sind. Durch die Anordnung der Befestigungselemente an den zueinander verschwenkbar gelagerten Haltern ist es möglich, die Orientierung und Positionierung der Befestigungselemente zueinander an den jeweiligen Haltern zu verändern, wobei die Veränderung innerhalb eines Verschwenkbereiches stattfindet, innerhalb dessen eine problemlose Befestigung von Komponenten an der orthopädietechnischen Einrichtung über die Befestigungselemente möglich ist. Beispielsweise können Antriebe, Führungen, Gelenke oder Dämpfer, die nur eine begrenzte Winkeltoleranz aufweisen, so einfach auf der Basislage des Grundkörpers vor dessen Fertigstellung positioniert werden, damit nach dem Fertigstellen der orthopädietechnischen Einrichtung beziehungsweise des Grundkörpers der orthopädietechnischen Einrichtung keine weiteren Zwischenstücke, Toleranzausgleiche oder Adapter vorgesehen werden müssen, um die Komponente an dem jeweiligen Befestigungselement festlegen zu können. Über die Positioniereinrichtung werden die Befestigungselemente auf der Basislage in den definierten Positionen zueinander angeordnet, wobei die Befestigungselemente eine Basis aufweisen können, von der zumindest ein Formschlusselement absteht. Über das Formschlusselement ist es möglich, weitere Komponenten wie Dämpfer, Gelenkeinrichtungen, Antriebe, Steuerungen oder dergleichen an dem Grundkörper festzulegen. Unter einem Formschlusselement wird auch eine Schraube oder eine Gewindestange verstanden. Durch das Anordnen der Befestigungselemente in den vorab definierten Positionen zueinander ist es möglich, aus einem Pool von Komponenten mit standardisierten Anschlusseinrichtungen auszuwählen, um diese dann auf der Basislage anzuordnen und auf dem Grundkörper festzulegen. Damit ist es beispielsweise möglich, Orthesenkomponenten individuell an den jeweiligen Patienten oder Orthesennutzer anzupassen und unterschiedliche Aktuatoren oder Gelenkeinrichtungen oder Korrektureinrichtungen an den Orthesenkomponenten anzuordnen, die sich beispielsweise an einen Genesungsfortschritt anpassen oder über die auf veränderte Umstände, beispielsweise Verschlimmerungen bei Erkrankungen reagiert werden kann. Darüber hinaus wird die Genauigkeit der Anordnung der Befestigungselemente auf dem Grundkörper erhöht.

Die Halter sind an einem Zentralstück schwenkbar gelagert. Dieses Zentralstück dient zur unabhängigen Verschwenkung der Halter zueinander. So ist es möglich, den einen Halter, beispielsweise einen distalen Halter, unabhängig von dem anderen Halter, einem proximalen Halter, relativ zu dem Zentralstück zu verschwenken. Über das Zentralstück kann eine Grundausrichtung der Positioniereinrichtung an dem jeweiligen Grundkörper vorgenommen werden, sodass eine Relativverlagerung der Halter zu dem Zentralstück und zueinander an dem jeweils vorgegebenen, eingeschränkten Winkelbereich um die Schwenkachse möglich ist. An dem Zentralstück wird die Schwenkachse für den ersten und zweiten Halter ausgebildet. Die Halter sind an dem Zentralstück vorteilhafterweise in einer einmal gefundenen Stellung fixierbar. Dies kann beispielsweise durch Festklemmen des Halters an dem Zentralstück erfolgen. Die Halter sind bevorzugt stufenlos innerhalb des Winkelbereiches verschwenkbar und in jeder Stellung zueinander und zu dem Zentralkörper an diesem fixierbar.

Um die Positioniereinrichtung an dem Grundkörper auszurichten und gegebenenfalls festlegen zu können, ist an dem Zentralstück eine Fixiereinrichtung zur Orientierung der gesamten Positioniereinrichtung auf dem Grundkörper angeordnet. Die Fixiereinrichtung kann ein Zapfen, ein Gewinde oder eine andere, vorzugsweise formschlüssige oder kraftschlüssige Festlegungseinrichtung sein. Grundsätzlich ist es auch möglich, dass über eine kraftschlüssige Fixiereinrichtung, beispielsweise eine Magneteinrichtung oder eine Kombination mehrerer Magneteinrichtungen an dem Zentralstück, eine Fixierung des Zentralstücks und damit der Positioniereinrichtung auf oder an dem Grundkörper erfolgt.

Jeder Halter der Positioniereinrichtung kann mehrere Aufnahmeeinrichtungen für jeweils ein Befestigungselement aufweisen, sodass beispielsweise an einer distalen Komponente der orthopädietechnischen Einrichtung zwei oder mehr Befestigungselemente in einer definierten Zuordnung zueinander positioniert werden können und an einer proximalen Komponente der orthopädietechnischen Einrichtung ebenfalls mehrere Befestigungselemente, beispielsweise drei Befestigungselemente, in einer definierten Zuordnung zueinander auf der Basislage des Grundkörpers ausgerichtet werden können. Werden mehrere Befestigungselemente auf den Orthesenkomponenten angeordnet, kann eine drehstarre Befestigung der jeweiligen zusätzlichen Einrichtung wie Aktuator, Dämpfer oder Gelenk erfolgen.

Die Aufnahmeeinrichtungen können Anlageflächen für das jeweilige Befestigungselement aufweisen, die an einem Halter in einer gemeinsamen Ebene liegen. Dadurch wird gewährleistet, dass alle Befestigungselemente an einem Halter in einer gemeinsamen Ebene oder auf einem gemeinsamen Niveau liegen und nur gemeinsam mit dem Halter relativ zu dem anderen Halter und damit zu der Anordnungsebene der anderen Befestigungselemente verkippt werden können. Die Ausgestaltung von Anlageflächen in einer Ebene an einem Halter für eine einheitliche Ausrichtung der daran befindlichen Befestigungselemente erleichtert eine Standardisierung der zusätzlichen Komponenten wie Gelenkeinrichtungen, Aktuatoren oder Dämpfer sowie die Fertigung. Grundsätzlich ist es auch möglich, alle Befestigungselemente an einem Halter auf unterschiedlichen Niveaus oder in unterschiedlichen Ebenen anzuordnen, wobei die Ebenen bevorzugt parallel zueinander ausgerichtet sind, jedoch auch verkippt zueinander sein können. Die Anordnung in einer Ebene erleichtert jedoch sowohl die Fertigung der Positioniereinrichtung als auch die Montage der Befestigungselemente und der daran festzulegenden Bauteile.

Die Aufnahmeeinrichtungen können als Hülsen ausgebildet sein, um die Befestigungselemente, die beispielsweise Zapfen, Innengewinde, Schäfte oder ähnliche Formschlusselemente oder Einrichtungen zur Festlegung der weiteren Komponenten aufweisen, aufnehmen zu können. Die Hülsen sind bevorzugt zylindrisch ausgebildet, grundsätzlich können auch andere Formgebungen, insbesondere ovale oder eckige Formen, vorgesehen sein.

Die Fixiereinrichtung ist in einer Weiterbildung der Erfindung so ausgebildet, dass die Schwenkachse der beiden Halter orthogonal zu einer Gelenkachse einer an dem Grundkörper anordenbaren Gelenkeinrichtung ausrichtet. Die Gelenkeinrichtung hat eine Gelenkachse, die sich an der physiologischen Gelenkachse orientiert und entsprechend ausgebildet und orientiert ist, im besten Fall mit der physiologischen Gelenkachse zusammenfällt. Die Gelenkeinrichtung ist beispielsweise ein Prothesengelenk oder ein Orthesengelenk. Fällt die Schwenkachse der Positioniereinrichtung mit der Gelenkachse der Gelenkeinrichtung zusammen, wird durch die Verschwenkbarkeit der Halter zueinander um die Schwenkachse die Individualisierung an den jeweiligen Grundkörper oder einen jeweiligen Patienten oder an die jeweilige Einrichtung ermöglicht. Ist beispielsweise der Grundkörper eine Orthese oder eine Vorstufe zu einer Orthese, kann der Grundkörper an die individuelle Körperform des Patienten angepasst werden. Wird die Basislage beispielsweise gelenkübergreifend an einer proximalen und distalen Gliedmaße angeordnet, beispielsweise dem Oberschenkel und dem Unterschenkel eines Patienten, ergibt sich aufgrund der unterschiedlichen anatomischen Gegebenheiten eine große Anzahl möglicher Orientierungen und Ausrichtungen von Orthesenkomponenten proximal und distal der natürlichen Gelenkachse. Die Längsachse der Fixiereinrichtung symbolisiert oder entspricht dann die Gelenkachse der Gelenkeinrichtung, die später an dem Grundkörper befestigt werden soll. Der begrenzte Winkelbereich an der Positioniereinrichtung, um den die Halter verschwenkt werden können, bildet die Fertigungstoleranzen oder Montagetoleranzen der Gelenkeinrichtungen ab, sodass bereits bei der Fertigung des Grundkörpers die Montagetoleranzen der jeweiligen Gelenkeinrichtung berücksichtigt werden können.

An dem Zentralstück kann ein Anschlagelement angeordnet sein, das mit einem an dem jeweiligen Halter angeordneten Gegenstück den Winkelbereich begrenzt. Der Winkelbereich, um den die Halter um eine Schwenkachse verschwenkbar sind, ist somit einstellbar oder veränderbar, beispielsweise um unterschiedliche Montagetoleranzen in der jeweils vorgesehenen Komponente oder der Komponenten berücksichtigen zu können. Werden winkeltolerante Gelenkeinrichtungen, Aktuatoren oder Dämpfer eingesetzt, kann der Winkelbereich größer gewählt werden, werden hinsichtlich der Verkippung der Lagerstellen empfindliche Komponenten vorgesehen, kann der eingeschränkte Winkelbereich verkleinert und auf das jeweils zulässige Maß reduziert werden.

Das Anschlagelement und/oder das Gegenstück können verstellbar an dem Zentralstück oder dem Halter angeordnet sein. Es kann für jeden Halter ein Anschlagelement oder ein Gegenstück vorgesehen sein, sodass eine individuelle Einstellung des jeweiligen Halters über die Verstellung des Anschlagelementes oder des Gegenstückes möglich ist. Ebenso ist es möglich, dass nur ein Anschlagelement vorgesehen ist, über das beide Halter hinsichtlich ihres Einstellbereiches veränderbar sind.

Eine Variante der Erfindung sieht vor, dass das Anschlagelement entlang der Schwenkachse verschieblich und in der jeweils gewünschten Position festlegbar an dem Zentralstück angeordnet ist. Durch die verschiebliche Anordnung des Anschlagelementes entlang der Schwenkachse ist es möglich, nach der Anordnung des Zentralstückes an dem Grundkörper und gegebenenfalls dessen Fixierung an dem Grundkörper zunächst einen Halter und die daran angeordneten Befestigungselemente auf der Basislage des Grundkörpers zu positionieren. In der dann gefundenen Position wird der Halter fixiert. Die Stellung des einen Halters begrenzt den Verschiebeweg des Anschlagelementes und legt die erreichbare Maximalposition des Anschlagelementes zu dem anderen Halter fest. Über die Position des Anschlagelementes an dem Zentralstück wird somit der verbleibende Verstellbereich oder Verschwenkbereich des anderen Halters relativ zu dem ersten Halter definiert. Wird eine Stellung der Halter zur optimalen Ausrichtung der Befestigungselemente auf oder an dem Grundkörper innerhalb des maximalen Winkelbereiches gefunden, werden die Halter an dem Zentralstück fixiert. Anschließend werden die Befestigungselemente an der Basislage festgelegt, beispielsweise über einen Kleber, eine Spachtelmasse, eine Ausgleichsmasse oder über Adapter. Die Positioniereinrichtung bietet somit die Möglichkeit, über einen festgelegten Toleranzbereich der Winkelausrichtungen der Befestigungselemente zueinander eine optimierte Anordnung der Befestigungselemente auf der Basislage, beispielsweise einer Prothese oder Orthese oder einer anderen orthopädietechnischen Einrichtung, zu ermöglichen. Der maximale Winkelverstellbereich um die Schwenkachse ist konstruktiv begrenzt. Das Anschlagelement stellt eine Kopplung zwischen den Haltern dergestalt her, dass die Verschwenkung des einen Halters um einen Verstellwinkel von dem möglichen Verstellwinkel des anderen Halters in entgegengesetzte Richtung abgezogen wird. Steht ausgehend von der Ausgangsstellung beiden Halteren ein Verschwenkbereich von +-5° zur Verfügung und wird der erste Halter bei einer waagerechten Schwenkachse um 3° nach oben verschwenkt, steht dem anderen Halter ein Verschwenkbereich von 2° nach oben und 8° nach unten zur Verfügung. Bei einer Anordnung der Positioniereinrichtung lateral an einem Träger wäre dies an dem ersten Halter eine Verschwenkung in Lateralrichtung um 3°, was für den anderen Halter eine maximale Verschwenkung um 2° in Lateralrichtung oder 8° in Medialrichtung bedeuten würde.

Sind trotz der Verschwenkung der Halter innerhalb des maximal zulässigen Winkelbereiches Ausgleichsmaßnahmen zu treffen, beispielsweise weil die Zuordnung einer Orthesenkomponenten für einen Oberschenkel zu einer Orthesenkomponenten für einen Unterschenkel eine vollflächige optimierte Auflage auf der Basislage nicht zulässt, wird der Freiraum ausgefüllt, beispielsweise über einen Kleber oder eine Spachtelmasse. Anschließend wird die orthopädietechnische Einrichtung nach der Fixierung der Befestigungselemente auf der Basislage und gegebenenfalls Auflage weiterer Decklagen und Abdecklagen, insbesondere aus faserverstärkten Verbundwerkstoffen, fertiggestellt. Dabei muss die Positioniereinrichtung nicht mehr mit den Befestigungselementen gekoppelt oder verbunden sein, vielmehr kann der Grundkörper zusammen mit den Befestigungselementen den weiteren notwendigen Verarbeitungsschritten zugeführt werden, beispielsweise unter hohen Temperaturen und im Vakuum ausgehärtet werden. Die Abwesenheit der Positioniereinrichtung, die bevorzugt aus einem metallischen Werkstoff besteht, ermöglicht eine Fertigung frei von Differenzen der thermischen Längenausdehnung, sodass eine präzisere Fertigung der orthopädietechnischen Einrichtung erfolgen kann.

Das Anschlagelement und/oder das Gegenstück können Anlageflächen aufweisen, die schräg oder gekrümmt zur Schwenkachse ausgerichtet sind. Über die schrägen oder gekrümmten Anlageflächen ist es möglich, eine stufenlose Anpassung der jeweiligen Verschwenkbereiche oder Winkelbereiche, um diejenigen Halter um die Schwenkachse verschwenkbar sind, zu erreichen. Durch die schrägen oder gekrümmten Anlageflächen wird bei einer verschieblichen Lagerung des Anschlagelementes bei einer Verdrehung eines Halters relativ zu dem anderen Halter eine Kraftkomponente in Verschieberichtung ausgeübt. Wenn der erste Halter maximal aus der Ausgangsstellung verschwenkt wird, wird das Anschlagelement von dem Gegenstück des ersten Halters in Richtung auf das gegenüberliegende Gegenstück verschoben und begrenzt dadurch dessen möglichen Verschwenkwinkel, ggf. bis auf Null.

Eine Weiterbildung der Erfindung sieht vor, dass das jeweilige Anschlagelement und das jeweilige Gegenstück korrespondierend ausgebildete, aufeinander zulaufend orientierte, insbesondere keilförmig orientierte Anlageflächen aufweisen, sodass bei Erreichen einer Endstellung des Anschlagelementes eine Grundstellung für den jeweiligen Halter relativ zu dem Zentralstück erreicht und definiert wird, während für den anderen Halter eine maximale Verschwenkbarkeit um die Schwenkachse in beiden Verschwenkrichtungen möglich ist. Befindet sich das Anschlagelement in einer Mittelstellung, sind beide Halter relativ zu dem Zentralstück in beide Richtungen um die Schwenkachse verschwenkbar gelagert. Der Winkelbereich der Verschwenkung pro Halter ist dann entsprechend halbiert. Dies ist insbesondere dann der Fall, um das Anschlagelement aneinander entgegengesetzten Enden je einen keilförmigen oder zum Ende aufeinander zu laufend orientierten Endbereich aufweist, die einem jeweils korrespondierend geformten Gegenstück zugeordnet ist. Bei einer asymmetrischen Ausgestaltung des Anschlagelementes können unterschiedliche Verschwenkbereiche je Stellung des Anschlagelementes dem einen oder anderen Halter zugeordnet werden.

Die Halter können in einem Winkelbereich von +- 10° um die Ausgangstellung verschwenkbar aneinander gelagert sein, wobei die Winkelbereiche für jeden Halter unterschiedlich ausgebildet sein können. So kann ein Halter einen Winkelbereich von +- 10° aufweisen, während der andere Halter eine Verschwenkung und einen Winkelbereich von +- 5° um die Ausgangsstellung aufweist. Dies wird durch die Ausgestaltung von Anschlagelement und Gegenstück definiert.

In einer Weiterbildung der Erfindung ist vorgesehen, dass die Aufnahmeeinrichtungen für die Befestigungselemente in der Ausgangsstellung der Halter zueinander eine parallel zueinander ausgerichtete Längserstreckung aufweisen. Dadurch wird gewährleistet, dass beispielsweise bei Gelenkeinrichtungen die Befestigung an den Befestigungselementen achsparallel erfolgen kann, sodass bei der Fertigung der Gelenkeinrichtung auch die Bohrungen, Hülsen oder Aufnahmen einfach achsparallel zu fertigen sind.

Ein System aus einer Positioniereinrichtung, wie sie oben beschrieben worden ist, und einem Grundkörper einer orthopädietechnischen Einrichtung sieht vor, dass der Grundkörper ein natürliches Gelenk einer Gliedmaße übergreifend und einstückig zur Anlage an der Gliedmaße ausgebildet ist. Die orthopädietechnische Einrichtung ist dabei bevorzugt als Orthese oder Prothese ausgebildet. Wenn der Grundkörper ein natürliches Gelenk einer Gliedmaße übergreifend und einstückig zur Anlage an der Gliedmaße ausgebildet ist, beinhaltet dies auch, dass eine fiktive Gliedmaße bei einer fehlenden distalen Gliedmaße eingesetzt werden kann. Die fehlende Gliedmaße kann dann anmodelliert werden, beispielsweise indem ein Gliedmaßenstumpf optisch erfasst wird, in einer 3D-Simulation bearbeitet und dann über ein 3D-Druckverfahren hergestellt wird. Nach Positionierung der Befestigungselemente auf dem Grundkörper und nach der Fixierung der Befestigungselemente auf dem Grundkörper sowohl auf der proximalen als auch auf der distalen Seite der Gelenkachse eines natürlichen Gelenkes findet dann gemeinsam eine Fertigstellung statt. Während und nach der Fertigstellung behalten die Befestigungselemente die durch die Positioniereinrichtung vorgegebenen Positionen, Orientierungen und Abstände bei. Anschließend kann der Grundkörper in eine distale und eine proximale Komponente aufgeteilt werden. Bei einer Ausgestaltung als Orthese hat man dann eine distale Orthesenschale und eine proximale Orthesenschale, bei einer Prothese hat man einen Prothesenschaft zur Aufnahme eines Stumpfes und eine Prothesenkomponente, die über eine Gelenkeinrichtung oder eine andere Befestigungseinrichtung, die an den Befestigungselementen festgelegt wird, an dem Prothesenschaft fixiert wird.

An dem Grundkörper kann eine Aufnahme zur Fixierung der Positionierungseinrichtung angeordnet sein. Die Aufnahme kann eine formschlüssige oder eine kraftschlüssige Aufnahme sein. Beispielsweise kann eine Setzmutter oder eine Setzschraube in dem Grundkörper oder an dem Grundkörper festgelegt sein, um dort die Positioniereinrichtung zu fixieren. Alternativ kann dies über eine magnetische Kopplung erfolgen. Die Aufnahme kann auch an oder in einem Träger angeordnet sein, auf den die Basislage des Grundkörpers aufgelegt und angeformt wird. Die Aufnahme muss nicht unlösbar mit der Basislage verbunden sein, es reicht eine stabil geometrische Zuordnung, so dass die Fixiereinrichtung bevorzugt entlang der Gelenkachse orientiert wird, damit die Schwenkachse orthogonal zu der Gelenkachse ausgerichtet werden kann.

Die Aufnahme kann in dem Bereich der Gelenkachse des natürlichen Gelenkes der Gliedmaße angeordnet sein, an der die orthopädietechnische Einrichtung, insbesondere Orthese oder Prothese, anordenbar ist. Fällt die Längserstreckung der Aufnahme mit der Schwenkachse der Halter zusammen, kann dies leicht durch eine Steckverbindung oder Schraubverbindung realisiert werden. Die Positioniereinrichtung wird dann auf die Aufnahme aufgesteckt oder in die Aufnahme eingeschraubt. Bei einer magnetischen Ausrichtung erfolgt die Ausrichtung der Positioniereinrichtung über die Orientierung der Magnete und der ferromagnetischen Elemente als Gegenstück.

Der Grundkörper kann an einem Modell der Gliedmaße oder an der Gliedmaße selbst angeformt sein und eine Kontur der Gliedmaße entsprechende Innenkontur aufweisen. Auf der Basislage des Grundkörpers wird das jeweilige Befestigungselement aufgesetzt, festgelegt, gegebenenfalls mit einer oder mehreren Decklagen abgedeckt und dann finalisiert.

An dem Grundkörper kann ein Solltrennbereich oder eine Solltrennstelle ausgebildet sein, entlang der der Grundkörper in eine proximale und eine distale Komponente teilbar ist. Der Solltrennbereich kann durch Materialschwächungen oder durch Weglassen von Faserverbundwerkstofflagen in diesem Bereich ausgebildet werden. In dem Solltrennbereich befindet sich dann nur die Basislage, die auch aus mehreren Lagen eines Faserverbundwerkstoffes bestehen kann.

Nachfolgend wird ein Ausführungsbeispiel der Erfindung anhand der beigefügten Figuren näher erläutert. Es zeigen: Nachfolgend wird ein Ausführungsbeispiel der Erfindung anhand der beigefügten Figuren näher erläutert. Es zeigen:
- Figuren 1a bis 1d: Ansichten eines Befestigungselementes;
- Figur 2: eine schematische perspektivische Ansicht eines Trägers mit einer aufgelegten Basislage;
- Figur 3: eine Basislage gemäß Figur 2 mit aufgesetzten Befestigungselementen;
- Figur 4: eine schematische Seitenansicht mit aufgelegter Faserverbundwerkstofflage;
- Figur 5: eine Schnittansicht gemäß Figur 2;
- Figur 6: eine Schnittansicht gemäß Figur 3;
- Figur 7: eine Schnittansicht gemäß Figur 4;
- Figur 8: eine Darstellung einer fertiggestellten Orthese;
- Figur 9: eine Positioniereinrichtung für die Befestigungselemente;
- Figur 10: die Positioniereinrichtung gemäß Figur 9 in Untenansicht;
- Figur 11: eine Explosionsdarstellung der Positioniereinrichtung;
- Figuren 12 und 13: Darstellungen von Haltern in unterschiedlichen Winkelstellungen,
- Figuren 14 und 15: Schnittdarstellungen der Figuren 12 und 13; sowie
- Figur 16: eine Variante des Zentralstückes.

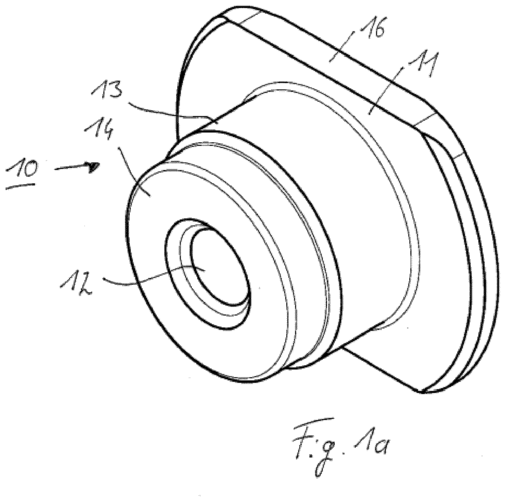

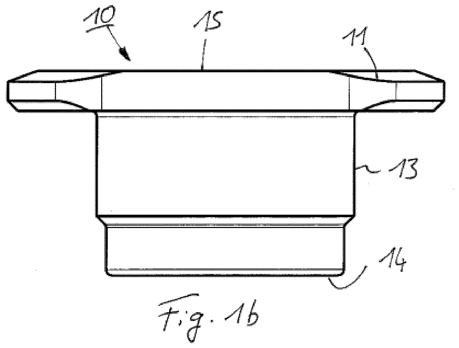

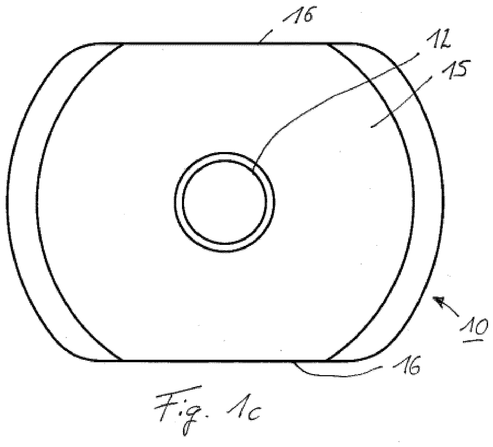

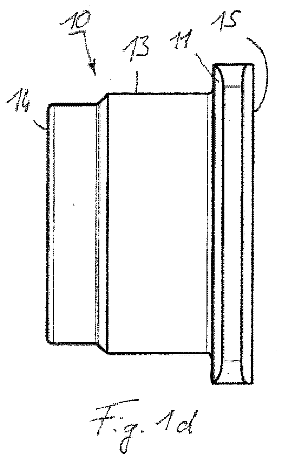

Figuren 1a bis 1d zeigen unterschiedliche Ansichten eines Befestigungselementes 10, wobei die Figur 1a eine perspektivische Gesamtansicht, die Figur 1b eine Seitenansicht, Figur 1c eine Untenansicht sowie Figur 1d eine weitere Seitenansicht sind. Das Befestigungselement 10 weist eine Basis 11 auf, die im dargestellten Ausführungsbeispiel im Wesentlichen flach und plattenförmig ausgebildet ist. An den Rändern der Basis 11 sind Abschrägungen ausgebildet, um eine verbesserte Anlage an eine Unterlage oder eine Auflage zur Ausbildung eines glatten Überganges zu bieten. Darüber hinaus kann zwischen den Abschrägungen und Faserverbindwerkstoffen Verbindungsmaterial oder ein Klebstoff angeordnet werden, um das Befestigungselement 10 daran zu fixieren. Die Basis 11 ist unrund ausgebildet und weist zwei Abflachungen 16 an einander gegenüberliegenden Seiten auf. Zwischen den Abflachungen 16 bildet die Basis 11 einen Radius aus, dessen Fortführung zu einer Kreisform führen würde. Die Kontur der Basis 11 entspricht also einem Kreis mit abgeschnittenen Kreissegmenten mit parallelen Kreissehnen. In der Mitte der Basis 11 ist eine Zentralbohrung mit einem Formschlusselement 12 in Gestalt eines Innengewindes ausgebildet. Das Innengewinde 12 erstreckt sich entlang der Längserstreckung eines Schaftes 13, der von der Basis 11 abragt. An der der Basis 11 entfernten Seite des Schaftes 13 ist eine Anbindungsfläche 14 ausgebildet, die im Wesentlichen eben ausgebildet ist. Der Anbindungsfläche 14 gegenüberliegend ist eine Bodenfläche 15 an der Basis 11 ausgebildet, die Anbindungsfläche 11 und die Bodenfläche 15 sind im Wesentlichen parallel zueinander orientiert. Der Schaft 13 ist rotationssymmetrisch, die Zentralbohrung mit dem Innengewinde 12 ist koaxial zur Längserstreckung des Schaftes 13 ausgebildet. Der Schaft 13 ist in dem vorderen Drittel, das zu der Anbindungsfläche 10 gerichtet ist, abgesetzt, das heißt, dass der Schaft 13 dort einen geringeren Durchmesser als im Bereich der Basis 11 aufweist. Die Größe des Absatzes kann variieren, insbesondere ist der Absatz so gewählt, dass auf die Basis 11 aufgelegte Lagen eines Faserverbundwerkstoffes bis zu diesem Absatz reichen oder aber zumindest nicht über den Absatz hinaus in Richtung auf die Anbindungsfläche reichen und gelegt werden. Die Außenkontur des Schaftes 13 kann auch andere Außenkonturen aufweisen, insbesondere eine nicht rotationssymmetrische Außenkontur, um eine Verdrehsicherung zusätzlich zu einer Verdrehsicherung über die unrunde Ausgestaltung der Basis 11 zu ermöglichen.

An dem Befestigungselement 10 können zudem Ausnehmungen, Vorsprünge oder Hinterschnitte angeordnet oder ausgebildet sein, um eine weitere Festlegung auf einer Basislage zur Herstellung eines Grundkörpers für eine orthopädietechnische Einrichtung auszubilden. Die Verwendung des Befestigungselementes 10 im Zusammenhang mit der Herstellung von orthopädietechnischen Einrichtungen wie Orthesen, Prothesen oder anderen orthopädietechnischen Komponenten wird nachfolgend erläutert. Die Basis 11 dient zur Festlegung des Befestigungselementes 10 an einem Grundkörper, während das Formschlusselement 12 dazu dient, dass an dem Befestigungselement 10 weitere Komponenten einer orthopädietechnischen Einrichtung festgelegt werden können, beispielsweise Gelenke, Aktuatoren, Dämpfer oder andere Einrichtungen oder Komponente.

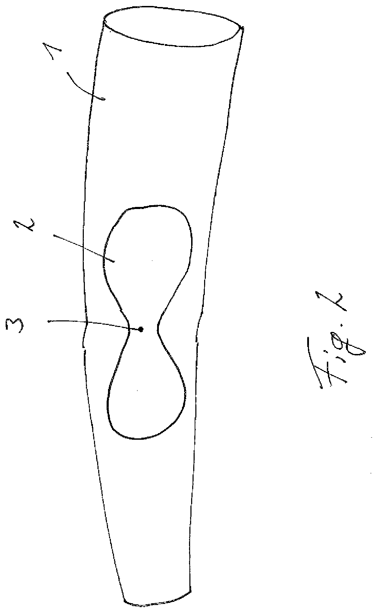

Die Herstellung einer Orthese als eine orthopädietechnische Einrichtung wird anhand der Figuren 2 bis 4 näher erläutert.

Figur 2 zeigt in schematischer Darstellung einen Träger 1, der korrespondierend zu dem Körperteil geformt ist, an dem eine Orthese oder Prothese getragen werden soll. Im dargestellten Ausführungsbeispiel ist der Träger 1 als ein Teil eines Beines mit einem Oberschenkelabschnitt, im Kniegelenk und einem Unterschenkelabschnitt ausgebildet. Alternativ zu einer Ausgestaltung in Beinform kann der Träger auch in Form eines Armes oder eines Armteils ausgebildet sein. Auch ist es möglich und vorgesehen, den Träger 1 in jeglicher anderer Form auszubilden, die notwendig ist, um eine Orthese auszubilden. Sollte eine Prothese hergestellt werden, kann der Träger 1 nur teilweise der Körperform des Patienten oder Prothesennutzers entsprechen, nämlich dort, wo der Stumpf noch vorhanden ist. Der distale Teil des Trägers wird dann modelliert, beispielsweise über ein 3D-Computerverfahren oder auf eine andere Art und Weise.

Auf den Träger 1 wird eine Basislage 2 aufgelegt, die aus einem oder mehreren Zuschnitten gebildet ist. Die Basislage 2 ist bevorzugt aus einem Faserverbundwerkstoff ausgebildet, beispielsweise aus einem Prepreg oder aus einem anderen Faserverbundwerkstoff. Die Basislage 2 im dargestellten Ausführungsbeispiel ist einteilig ausgebildet und erstreckt sich über eine Gelenkachse 3 eines natürlichen oder angenommenen Gelenkes der jeweiligen Gliedmaße. Im dargestellten Ausführungsbeispiel mit dem Träger 1 als Oberschenkelkeil überdeckt die Basislage 2 die Kniegelenkachse 3. Die Basislage 2 ist ausreichend flexibel, um sich an die Oberflächenkontur des Trägers 1 anpassen zu können. Der Träger 1 kann gegenüber der tatsächlichen Kontur der Gliedmaße modifiziert sein, beispielsweise durch Materialzugaben, Glättungen eines 3D-Modells oder ähnliches, um beispielsweise Polsterelemente auf der Innenseite der zu fertigenden Orthese oder Prothese anordnen zu können. Bei einer Prothese kann es notwendig sein, den Prothesenschaft oder die Aufnahmeeinrichtung größer zu wählen, um Liner oder andere Schutzüberzüge aufnehmen zu können, ohne dass eine zu hohe Pressung auf das Körperteil ausgeübt wird.

Die Basislage 2 ist geschlossen ausgebildet, also für Komponenten wie die Befestigungselemente 10, die auf der Basislage 2 aufgelegt werden, undurchlässig. Die Basislage 2 kann auf dem Träger 1 fixiert werden, entweder mechanisch oder über einen Kleber. Die Fixierung erfolgt so, dass die Basislage 2 nach der Fertigstellung der Orthese oder Prothese wieder abnehmbar ist.

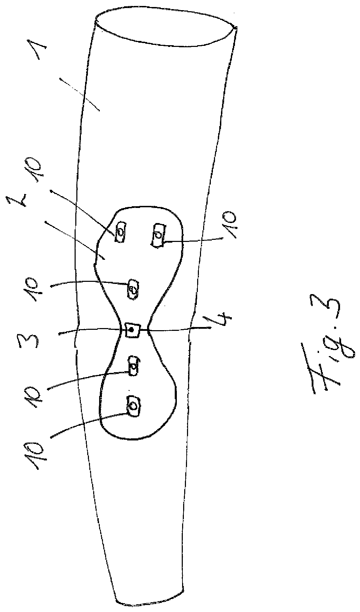

In der Figur 3 ist eine nächste Phase der Herstellung der Orthesenkomponenten gezeigt, bei der auf der lateralen Oberfläche der Basislage 2, also auf derjenigen Oberfläche, die dem Träger 1 abgewandt ist, Befestigungselemente 10 aufgesetzt sind, wie sie bereits in der Figur 1 beschrieben worden sind. Die Befestigungselemente 10 werden mit der Unterseite 15, also derjenigen Fläche der Basis 11, die der Anlagefläche 14 abgewandt ist, aufgesetzt. Dabei werden die Befestigungselemente 10, im dargestellten Ausführungsbeispiel fünf Befestigungselemente 10, von den zwei im distalen Bereich und drei im proximalen Bereich positioniert sind, bevorzugt über eine Positioniereinrichtung auf der Basislage 2 positioniert. Die Positioniereinrichtung wird weiter unten näher erläutert werden. Durch die Positioniereinrichtung sind die Befestigungselemente 10 in definierten Abständen zueinander und von der Gelenkachse 3 entfernt auf der Basislage 2 angeordnet. Die Positioniereinrichtung wird an einer Aufnahme 4 festgelegt oder darauf aufgesetzt, beispielsweise aufgesteckt, aufgeschraubt oder über eine Magnetriegelung fixiert. Die Aufnahme 4 ist bevorzugt bereits auf dem Träger 1 angeordnet und durchragt eine Ausnehmung in dem Zuschnitt der Basislage 2. Die Aufnahme 4 kann in dem Träger 1 eingearbeitet sein, beispielsweise eingegossen oder eingesetzt. Sie enthält bevorzugt ein Gewinde, eine Hülse oder einen Zapfen, deren Längserstreckung mit der Kniegelenksachse zusammenfällt. Allgemein gesprochen soll die Längserstreckung der Aufnahme 4 mit der Gelenkachse zusammenfallen, um die ein Orthesenoberteil gegenüber einem Orthesenunterteil oder eine Proximalkomponente gegenüber der Distalkomponente der Orthese verschwenkt.

Die Befestigungselemente 10 werden auf der Basislage 2 fixiert, beispielsweise über einen Kleber, eine Spachtelmasse oder aber auch unter Einsatz eines Ausgleichsmaterials. Angestrebt ist, dass sich das Befestigungsmittel wie Spachtel oder Kleber bei der weiteren Behandlung der Orthese nicht verformt. Zur Fertigstellung der Orthese kann diese unter hohen Temperaturen und unter Vakuum ausgehärtet werden, was nicht zu einer Verlagerung der Befestigungselemente 10 oder zu einer Verkippung der Befestigungselemente 10 führen darf.

Nachdem alle Befestigungselemente 10 auf der Basislage 2 befestigt sind, wird die Positioniereinrichtung entfernt, was später erläutert werden wird. Sowohl die Befestigungselemente 10 als auch die Aufnahmeeinrichtung 4 bleiben sicher auf der äußeren oder lateralen Oberfläche der Basislage 2.

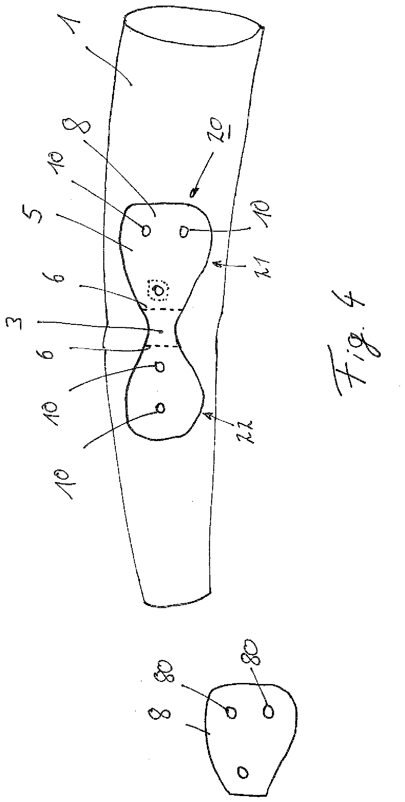

Anschließend wird zumindest eine Lage 8 eines Faserverbundwerkstoffes mit vorgestanzten Ausnehmungen über die Schäfte der Befestigungselemente 10 gelegt, wobei die Ausnehmungen in der Lage 8 aus einem Faserverbundwerkstoff so dimensioniert sind, dass der jeweilige Schaft hindurchragen kann, die Basis 11 jedoch nicht. Dadurch wird die Basis 11 des Befestigungselementes 10 zwischen der Basislage und einer äußeren Faserverbundwerkstofflage 8 eingebettet. In der äußeren Faserverbundwerkstofflage 8 können Solltrennlinien 6 eingearbeitet werden, entlang derer eine Trennung leicht oder erleichtert erfolgen kann. In dem dargestellten Ausführungsbeispiel wird durch zwei Solltrennlinien 6 ein Solltrennbereich ausgebildet, in dem die Gelenkachse 3 und auch die Aufnahmeeinrichtung 4 vor der Ankerplatte liegt. Nach dem Trennen an den Solltrennlinien 6 ergibt sich eine Proximalkomponente 21 und Distalkomponente 22 der Orthese, also eine Oberschenkelschale 21 und eine Unterschenkelschale 22, mit darin einlaminierten Befestigungselementen 10. Das Trennen oder Entfernen des Solltrennbereiches zwischen den Solltrennlinien 6 erfolgt erst, nachdem die Basislage 2 zusammen mit der zumindest einen Faserverbundwerkstofflage 8 auf der Außenseite verklebt und dann aufeinander festgelegt wurde. Dies geschieht beispielsweise nach Anlegen eines Unterdruckes in einem Ofen bei erhöhten Temperaturen. Die Faserverbundwerkstofflagen 8 werden bevorzugt bis zu dem Absatz in dem Schaft 13 aufgelegt. Über den Absatz wird gewährleistet, dass eine ausreichende Materialstärke in dem Bereich der Befestigungselemente vorhanden ist. Eine Faserverbundwerkstofflage 8 als Zuschnitt mit vorgefertigten Ausnehmungen 80, die in dem Durchmesser mit den Schaftdurchmessern der Schäfte 13 und in ihren Positionen den Positionen der Befestigungselemente 10 auf der Basislage 2 entsprechen, ist links in der Figur 4 gezeigt.

Nach dem Aushärten und Abkühlen des Laminatwerkstoffes ist ein Grundkörper 20 vorhanden, mit einer auf der Innenseite durchgehenden Basislage 2, darauf aufgesetzten Befestigungselementen 10 und zumindest einer, vorzugsweise mehreren Lagen Faserverbundwerkstoff 8, die miteinander verbunden sind, so dass die Befestigungselement 10 einlaminiert sind. Der Orthesengrundkörper 20 wird nach dem Aushärten und Abkühlen im Bereich der Solltrennstellen 6 getrennt, beispielsweise zersägt, um die Oberschenkelschale oder proximale Orthesenkomponente 21 von der Unterschenkelschale oder distalen Orthesenkomponente 22 zu trennen. Anschließend werden die Orthesenkomponenten 21, 22 von dem Träger 1 entfernt, gegebenenfalls nachbearbeitet und geschliffen, mit Aufnahmen für Befestigungseinrichtungen wie Gurten versehen und mit den notwendigen und dafür vorgesehenen Anbauteilen wie Gelenkeinrichtungen, Dämpfer oder Polsterungen ausgestattet.

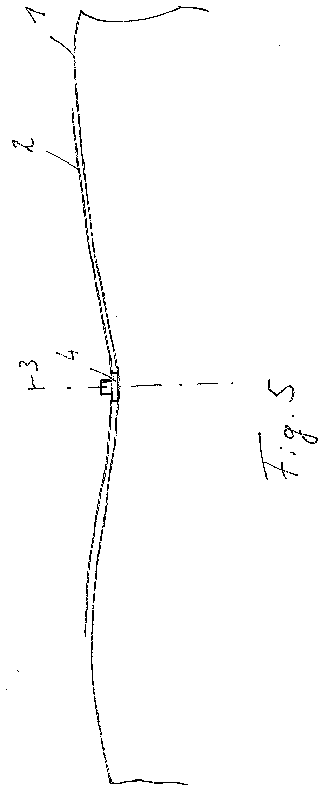

Figuren 5 bis 7 zeigen den Herstellungsablauf einer schematischen Schnittdarstellung. Zunächst wird auf dem Träger 1 die Ankerplatte oder die Aufnahme 4 positioniert, und zwar im Bereich der Gelenkachse des natürlichen Gelenkes oder einer Kompromissachse 3. Anschließend wird die Basislage 2 auf die äußere oder laterale Oberfläche des Trägers 1 gelegt und gegebenenfalls fixiert. Das Material der Basislage 2 kann plastisch verformbar sein und über geringe Rückstellkräfte verfügen, sodass ein möglichst vollständiges Anliegen an der äußeren Oberfläche des Trägers 1 ermöglicht wird. Der Abstand zu dem Träger 1 ist aus Gründen der besseren Sichtbarkeit eingezeichnet.

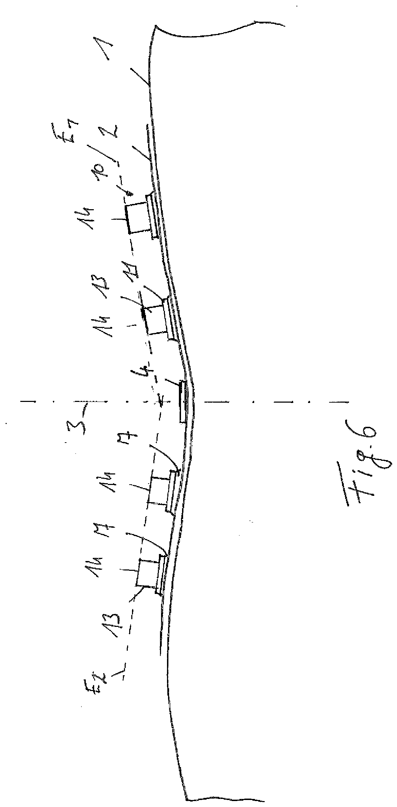

In der Figur 6 ist der Zustand nach dem Aufsetzen der Befestigungselemente 10 auf der lateralen Oberfläche der Basislage 2 gezeigt. Die Befestigungselemente 10 sind über eine Positioniereinrichtung zu der Gelenkachse 3 ausgerichtet auf der Basislage 2 positioniert. Es ist zu erkennen, dass die jeweiligen Basen 11 der Befestigungselemente 10 möglichst nahe an der Oberfläche der Basislage 2 angeordnet werden sollten. Die Verbindung der jeweiligen Unterseite 15 der jeweiligen Basis 11 der Befestigungselemente 10 erfolgt in dem dargestellten Ausführungsbeispiel über eine Spachtelmasse 7, die gleichzeitig Unebenheiten in der Oberfläche der Basislage 2 ausgleicht und dafür sorgt, dass die Befestigungselemente 10 fest auf der Basislage 2 verankert werden.

In Figur 6 ist zu erkennen, dass alle Anbindungsflächen 14 in jeweils einer Ebene E1, E2 liegen, wobei die Ebene E1 für die Befestigungselemente 10 der Proximalkomponente 21 und die Ebene E2 für die Befestigungselemente der Distalkomponente 22 steht. Der Figur 6 ist zu entnehmen, dass die Ebenen E1, E2 in dem dargestellten Ausführungsbeispiel nicht parallel zueinander liegen oder eine gemeinsame Ebene ausbilden. Dies wäre der Fall, wenn sich bei dem Ausführungsbeispiel beispielsweise ein vollständig gerades Bein an der lateralen Seite oder medialen Seite ergeben würde. Dargestellt ist eine mehr natürliche Darstellung, bei der sich eine laterale Auswölbung sowohl des Oberschenkels als auch des Unterschenkels ausgehend von dem Kniegelenk ergibt. Beide Ebenen E1, E2 schneiden sich in dem dargestellten Ausführungsbeispiel in der Gelenkachse 3, sodass sich eine gemeinsame Schnittlinie ergibt, die bevorzugt orthogonal auf der Gelenkachse 3 steht. Es ist auch möglich, dass die Anbindungsflächen nicht exakt in einer Ebene E1, E2 liegen, sondern ein gewisser Höhenversatz vorhanden ist. Ebenfalls ist es möglich, dass sich die Ebenen E1, E2 nicht in der Gelenkachse 3 schneiden, weil sich beispielsweise ein Höhenversatz eingestellt hat. Bevorzugt liegen alle Anbindungsflächen 14 aller Befestigungselemente 10 einer Orthesenkomponente 21, 22 auf einer gemeinsamen Ebene E1, E2. Die Längserstreckungen aller Bohrungen, Zapfen oder Formschlusselemente 12 wie Innengewinde oder Außengewinde in den Befestigungselementen 10 sind parallel bevorzugt zueinander ausgerichtet, bezogen jeweils auf eine Orthosenkomponente. Das heißt, dass alle Längsachsen der Befestigungselemente 10 auf der proximalen Orthesenkomponente 21 bevorzugt parallel zueinander ausgerichtet sind, ebenso wie die Längserstreckungen oder Längsachsen der Befestigungselemente 10 auf einer distalen Orthesenkomponente 22.

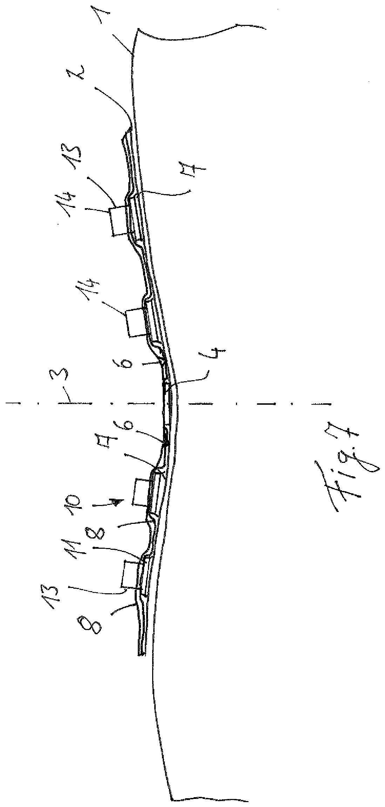

Nach der Festlegung der Befestigungselemente 10 auf der Basislage 2 werden, wie in der Figur 7 gezeigt, mehrere Lagen 8 eines Faserverbundwerkstoffes aufgelegt, beispielsweise harzgetränkte Fasermatten, gegebenenfalls unter Zugabe weiterer Kleber, Härter, Lösungsmittel oder dergleichen. Die Lagen 8 des Faserverbundwerkstoffes oder der Faserverbundwerkstoffe können in unterschiedlichen Orientierungen aufgelegt werden, um die Basen 11 der Befestigungselemente 10 einzulaminieren. Dazu sind in den Zuschnitten der Faserverbundwerkstofflagen 8 Ausnehmungen 80 oder Ausstanzungen ausgebildet, die der Form und dem Durchmesser der jeweiligen Schäfte 13 entsprechen. Da die Basen 11 größer als die Durchmesser der Schäfte 13 sind, kann nach dem Verbinden der Faserverbundwerkstofflagen 8 mit der Basislage 2 kein Befestigungselement 10 mehr aus der jeweiligen Orthesenkomponente 21, 22 entfernt werden. Aufgrund der unrunden Ausgestaltung der Basis 11 ist eine verdrehgesicherte Festlegung aller Befestigungselemente 10 gesichert. Um die Verdrehsicherung zu erhöhen, können Vorsprünge, Haken, Hinterschneidungen oder Ähnliches vorgesehen sein, damit die Befestigungselemente 10 sich nach der Fertigstellung der Orthesenkomponenten 21, 22 nicht verdrehen können.

Alle Anbindungsflächen 14 sind nicht von einer Faserverbundwerkstofflage 8 abgedeckt, um die Zugänglichkeit zu den Formschlusselementen 12 zu gewährleisten und eine definierte Anlage der zu montierenden Komponenten zu gewährleisten. Um Verunreinigungen des Formschlusselementes 12 zu vermeiden, kann diese gesichert werden. Bei der Ausführungsform gemäß der Figur 1, bei der das Formschlusselement 12 als ein Innengewinde ausgebildet ist, kann dieses beispielsweise über eine Schraube erreicht werden, die nach der Fertigstellung der Orthesenkomponente herausgeschraubt wird. Ist das Formschlusselement 12 als ein Außengewinde ausgebildet, kann eine Schraubkappe aufgeschraubt werden, um das Gewinde zu schützen. Korrespondierendes gilt für andere Formschlusselemente, wie Zapfen, Bohrungen oder dergleichen. Nach der Auflage der Faserverbundwerkstofflagen 8 wird unter Anlegung eines Vakuums und Aufbringung erhöhter Temperaturen auf dem Träger 1 der Orthesengrundkörper 20 fertiggestellt. Die Solltrennstellen 6 sind proximal und distal an der Gelenkachse 3 ausgebildet, beispielsweise durch Eindrückungen oder durch Schnitte in den Faserverbundwerkstofflagen 8 oder einfach durch keine oder weniger Faserverbundwerkstofflagen 8 in dem Bereich zwischen den Solltrennstellen 6.

Nach dem Aushärten und Trennen der Orthesenkomponenten 21, 22 voneinander können andere Komponenten an den Befestigungselementen 10 festgelegt werden.

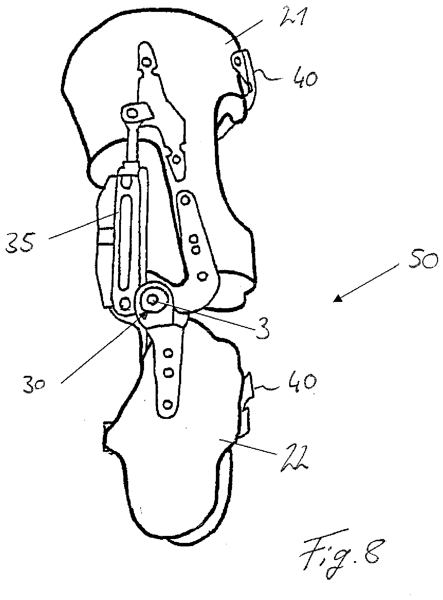

In der Figur 8 ist eine Variante einer Kniegelenksorthese gezeigt, bei der Proximalkomponente 21 als Oberschenkelschale und die Distalkomponente 22 als Unterschenkelschale ausgebildet ist. An beiden Orthesenkomponenten 21, 22, sind Befestigungseinrichtungen 40 angeordnet, die als Gurte ausgebildet sind, um Orthese 50 an einem Bein festzulegen. Eine Gelenkeinrichtung 30 mit einem hydraulischen Aktor 35 ist an den nicht mehr sichtbaren Befestigungselementen festgelegt, beispielsweise über Schrauben. Die Gelenkeinrichtung 30 hat ihre Schwenkachse im Bereich der Gelenkachse 3 des natürlichen Gelenkes. Die Position der Gelenkachse 3 auf der Gelenkeinrichtung 30 ist durch die exakte Positionierung der Befestigungselemente relativ zu der Gelenkachse 3 des natürlichen Gelenkes über eine Positioniereinrichtung gesichert. Die Form der Orthesenkomponenten 21, 22 in Gestalt der Orthesenschalen ist sehr gut und individuell an die Form des jeweiligen Orthesennutzers angepasst. Die Herstellung der Orthese kann ohne vorherige Anordnung der Gelenkeinrichtung 30 oder einer hydraulischen Komponente 35 an den Orthesenkomponenten 21, 22 erfolgen, was im Hinblick auf die bei der Fertigung auftretenden hohen Temperaturen und Unterdrücke insbesondere für elektronische Steuerungen außerordentlich vorteilhaft ist. Durch die Art und Weise der Fertigung wird eine Restriktion der zu verwendenden Anbauteile wie Dämpfer, Steuerung oder dergleichen verhindert.

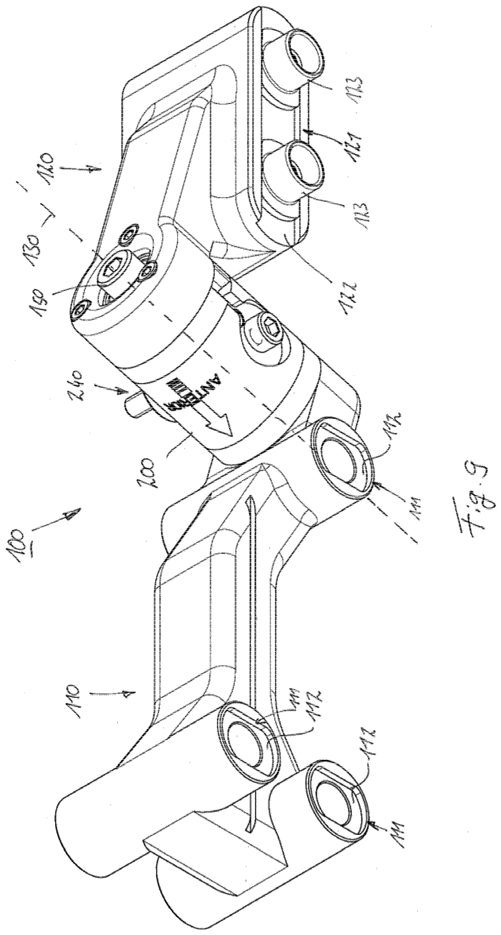

Figur 9 zeigt in einer perspektivischen Ansicht eine Positioniereinrichtung 100 zur Positionierung und Ausrichtung von nicht dargestellten Befestigungselementen 10, wie sie in der Figur 1 beispielhaft erläutert sind. Die Positioniereinrichtung 100 weist einen Zentralkörper 200 auf, an dem zwei Halter 110, 120 schwenkbar um eine Schwenkachse 130 angeordnet sind. Ein erster Halter 110 ist in dem dargestellten Ausführungsbeispiel für die Zuordnung und Anordnung der Befestigungselemente 10 auf der proximalen Orthesenkomponente 21 vorgesehen, während der zweite Halter 120 für die Befestigungselemente 10 auf der distalen Orthesenkomponente 22 vorgesehen ist. Beide Halter 110, 120 weisen Aufnahmeeinrichtungen 111, 121 auf, die als Hülsen ausgebildet sind, die Durchgangsbohrungen aufweisen, durch die Fixierelemente 123 hindurchführbar sind. In der Figur 9 sind die Fixierelemente 123 nur an dem zweiten Halter 120 gezeigt. An den Aufnahmeeinrichtungen 111, 121 sind Anlageflächen 112, 122 für die Oberseite der Basis 11 der Befestigungselemente 10 ausgebildet. Die Oberseite der Basis 11 ist diejenige Seite der Basis 11, die Unterseite 15 gegenüberliegt. In dem dargestellten Ausführungsbeispiel sind alle Anlagenflächen 112, 120 an einem gemeinsamen Halter 110, 120 in einer gemeinsamen Ebene angeordnet, um sicherzustellen, dass alle Befestigungselemente 10 in einer gemeinsamen Ebene liegen, wenn sie an dem jeweiligen Halter 110, 120 angeordnet und durch die Fixierelemente 123 dort festgelegt sind.

An dem Zentralkörper 200 ist eine Fixiereinrichtung 240 in Gestalt einer Schraube angeordnet, über die der Zentralkörper 200 an der Aufnahme 4, die an dem Träger 1 oder der Basislage 2 fixiert ist, festgelegt wird. Die Längserstreckung der Fixiereinrichtung 240 verläuft senkrecht zu der Schwenkachse 130 und schneidet diese vorzugsweise, sodass die Längsachse der Fixiereinrichtung 240 orthogonal auf der Schwenkachse 130 steht. Die Längserstreckung der Fixiereinrichtung 240 fluchtet bevorzugt mit der Längsachse 3 der Gelenkeinrichtung und der natürlichen Gelenkachse oder der Kompromissachse für das natürliche Gelenk. Befinden sich alle Anlageflächen 112, 122 in parallelen Ebenen oder in einer gemeinsamen Ebene, je nachdem, wie die Ebenen der Anlageflächen 112, 120 angeordnet sind, befindet sich die Positioniereinrichtung 100 in einer Ausgangsstellung. Aus dieser Ausgangsstellung können sowohl der erste Halter 110 als auch der zweite Halter 120 um einen eingeschränkten Winkelbereich, beispielsweise um +/- 10°, um die Schwenkachse 130 verschwenkt werden. Gelenkeinrichtungen 30 oder auch andere Anbauteile können hinsichtlich eines möglichen Winkelversatzes ihrer Anbindungsstellen empfindlich sein. Durch die Positioniereinrichtung 100 ist es möglich, neben einer exakten Positionierung der Befestigungselemente 10 relativ zueinander und zu einer Gelenkachse 3 um eine Gelenkeinrichtung 30 diesen maximalen Winkelversatz Rechnung zu tragen. Es besteht die Möglichkeit, den Winkelversatz der beiden Halter 110, 120 vorab größenmäßig festzulegen. Ist beispielsweise ein maximaler Versatz der Ebenen der Anbindungsflächen 14 von 10° zulässig, kann dieser maximale Winkelbereich mit der Positioniereinrichtung 100 eingestellt werden. Wird dann ausgehend von der Ausgangsstellung zunächst der erste Halter 110 auf die Basislage 2 aufgelegt und benötigt eine Verschwenkung in Lateralrichtung um 3°, ausgehend von der Ausgangsstellung, steht für den zweiten Halter 120 ein maximaler Verschwenkbereich um weitere 7° in Lateralrichtung zur Verfügung. Ist bei einer solchen Maximaleinstellung eine zufriedenstellende Orientierung der Unterseiten 15 der Basen 11 aller Befestigungselemente 10 nicht möglich, muss die gesamte Positioniereinrichtung 100 weiter lateral versetzt werden oder aber über eine Ausgleichsmasse oder über eine Spachtelmasse eine Festlegung der Befestigungselemente 10 auf der Basislage erfolgen.

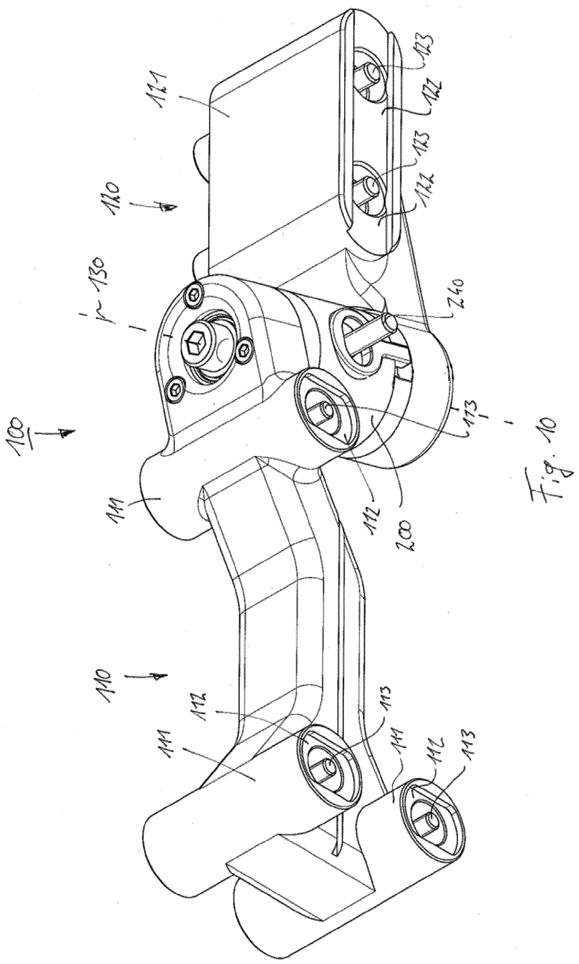

Die Positioniereinrichtung 100 ist klappsymmetrisch ausgebildet. In der Figur 9 ist beispielsweise eine Oberseite zu erkennen, während in der in Figur 10 die Unterseite dargestellt ist. Der Vergleich der Figuren 9 und 10 zeigt, dass auf beiden Seiten der Aufnahmeeinrichtungen 111, 121 gleiche Aufnahmen für die Befestigungselemente 10 ausgebildet sind. Die Fixiereinrichtung 240 kann aus dem Zentralkörper 200 herausgenommen und umgekehrt wieder eingeführt werden, sodass die Positioniereinrichtung 100 sowohl für ein rechtes Bein als auch für ein linkes Bein sowie für eine mediale als auch laterale Positionierung auf Basislagen 2 geeignet ist.

In der Figur 10 sind die Fixierelemente 113 in Gestalt von Schrauben in allen Aufnahmeeinrichtungen 111 gezeigt. Die Innengewinde 12 gemäß der Figur 1 sind korrespondierende zu Außengewinden an den Fixierelementen 113 ausgebildet, sodass die Montage dergestalt erfolgt, dass in jeder Aufnahmeeinrichtung 111 mit den Anbindungsflächen voran der Schaft 13 in die Bohrungen der hülsenartigen Aufnahmeeinrichtungen 111 eingeführt werden. Über die Fixierelemente 113 werden die Befestigungselemente fixiert. Es ist zu erkennen, dass die Form der Anlageflächen 112 der Aufnahmeeinrichtung 111 der Form und Kontur der Basen 11 entsprechen, sodass eine definierte Zuordnung und Orientierung jedes Befestigungselementes 10 an dem jeweiligen Halter 110, 120 erfolgt. In dem zweiten Halter 120 ist eine nutenartige Führung für die beiden Basen 11 der beiden Befestigungselemente 10 vorgesehen. Hierin können weitere Einlageelemente wie Schienen oder Verstärkungselement oder Abstandshalter aufgenommen werden, die ebenfalls einlaminiert werden können. Nach der Fixierung der Befestigungselemente 10 innerhalb der Aufnahmeeinrichtungen 111, 121 wird die Positioniereinrichtung 100 mit der Fixiereinrichtung 240 in der Aufnahme 4 festgelegt. Eine Zentralschraube 150 entlang der Schwenkachse 130 hält die beiden Halter 110, 120 einer definierten Stellung zueinander, bevorzugt in der Ausgangsstellung, bei der alle Unterseiten 15 der Befestigungselemente 10 in einer gemeinsamen Ebene oder zumindest in parallelen Ebenen zueinander ausgerichtet sind. Wird die Fixierung durch die Zentralschraube 150 gelöst, können die beiden Halter 110, 120 um die Schwenkachse 130 in dem vorgegebenen Winkelbereich verschwenken.

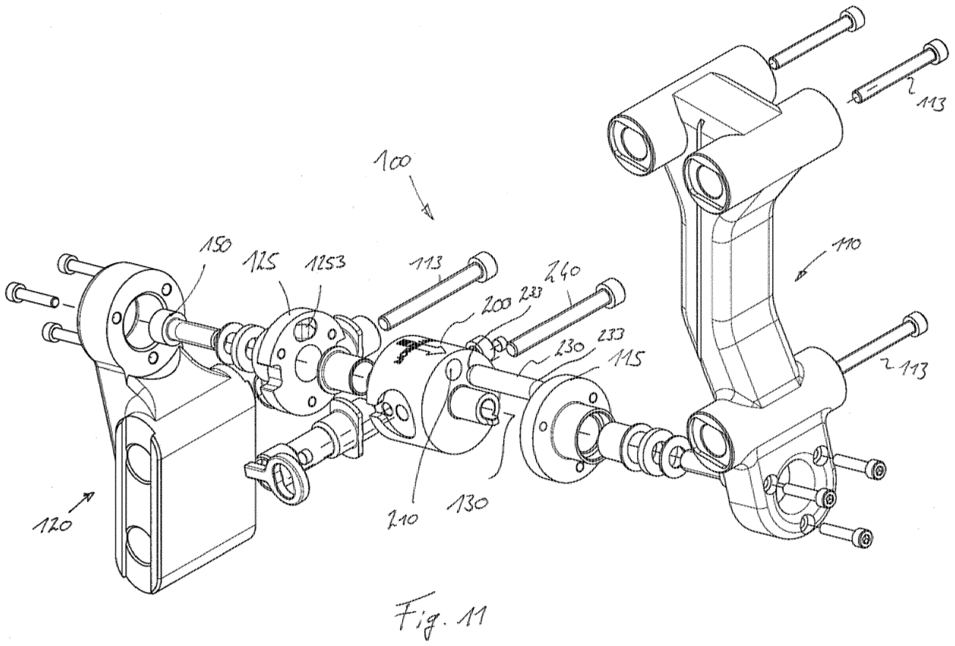

Figur 11 eine Explosionsdarstellung der Positioniereinrichtung 100 mit dem Zentralkörper 200, der Fixiereinrichtung 240, die durch eine Bohrung innerhalb des Zentralkörpers 200 hindurchgeht und die Schwenkachse 130 orthogonal schneidet. Die Fixierelemente 113 sind zu erkennen, ebenso die beiden Halter 110, 120 und die Zentralschraube 150, die sich entlang der Schwenkachse 130 erstreckt. Innerhalb des Zentralkörpers 200 ist ebenfalls ein Anschlagelement 230 in einer Bohrung 210 in dem Zentralkörper 200 längsverschieblich gelagert. Die Bohrung 210 erstreckt sich parallel zu der Schwenkachse 130.

An den Haltern 110, 120 sind über drei Schrauben jeweils ein Gegenstück 115, 120 mit Anlagenflächen 1153, 1253 angeordnet, die mit den Anlagenflächen 233 an den beiden Enden des Anschlagelementes 230 wechselwirken. Die Wechselwirkung wird nachfolgend erläutert. Die Gegenstücke 115, 125 sind in dem dargestellten Ausführungsbeispiel ortsfest an dem jeweiligen Halter 110, 120 gelagert.

Es besteht auch die Möglichkeit, beispielsweise durch Langlöcher eine Verdrehbarkeit der Gegenstücke 115, 125 an dem jeweiligen Halter 110, 120 zu ermöglichen. Über die Verdrehung der Gegenstücke 115, 125 kann eine Stellung des Winkelbereiches erfolgen, eine Vergrößerung des maximalen Winkelbereiches kann beispielsweise über einen Austausch der Gegenstücke 115, 125 erfolgen. Ebenfalls ist es möglich, beispielsweise durch Verstellschrauben die Position der Anschlagflächen 1153, 1253 zu verändern, um den Winkelbereich, um den der erste Halter 110 relativ zu dem zweiten Halter 120 um die Schwenkachse 130 verschwenkt werden kann, einzustellen. Dazu können Verstellschrauben in die Ausnehmungen in dem Gegenstück 115, 125 eingeschraubt oder herausgeschraubt werden.

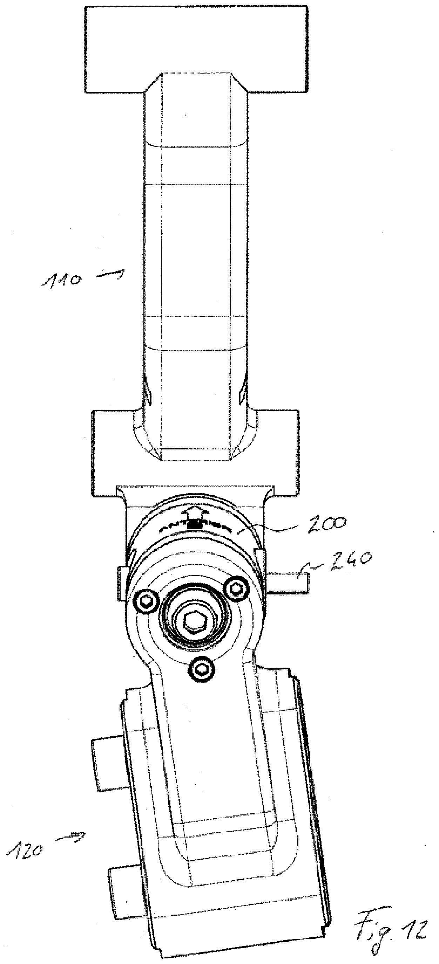

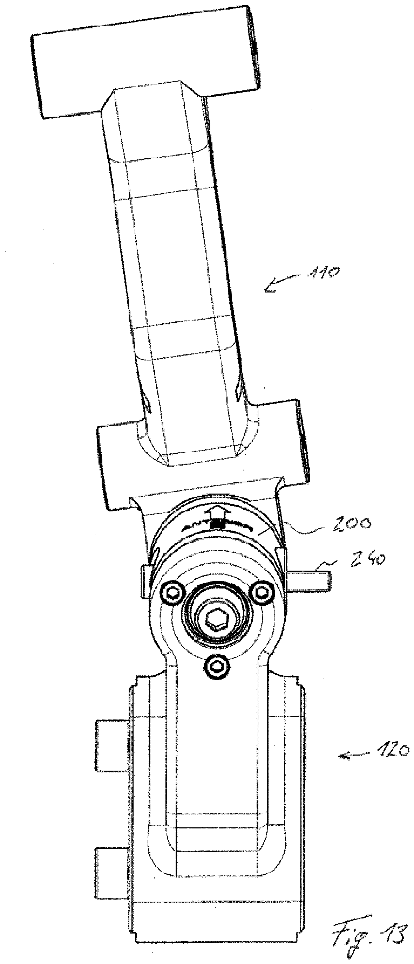

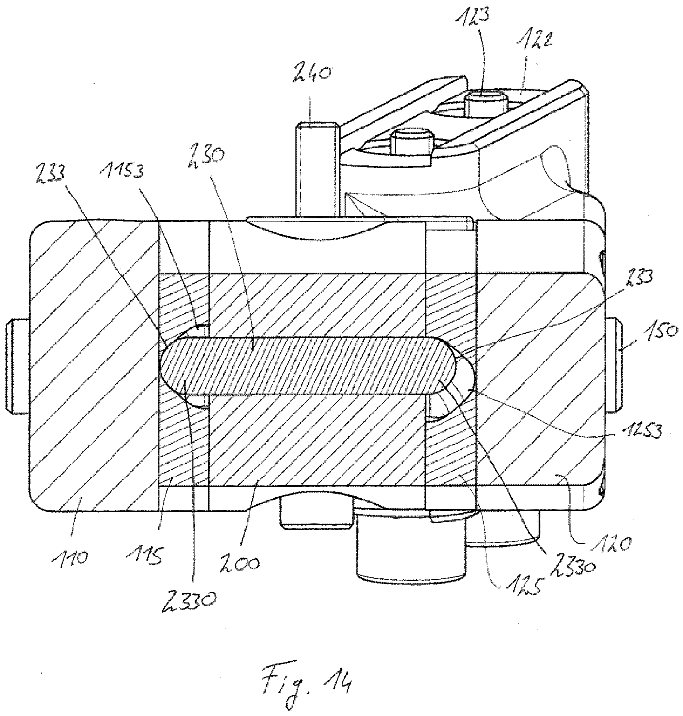

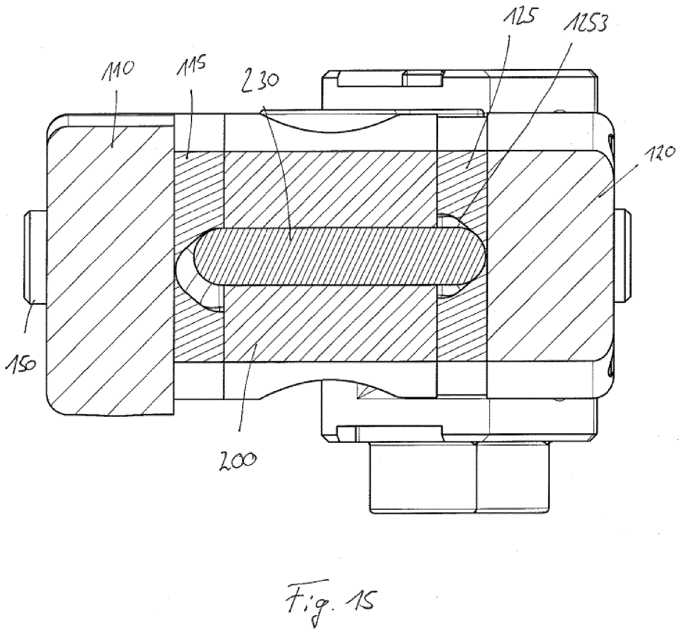

Figuren 12 und 13 zeigen die Positioniereinrichtung 100 in der jeweils gleichen Ansicht. In der Figur 12 ist ausgehend von der Ausgangsstellung der zweite Halter 120 maximal entgegen dem Uhrzeigersinn um die Schwenkachse 130 verschwenkt. In der Figur 13 ist der erste Halter 110 maximal entgegen dem Uhrzeigersinn, ausgehend von der Ausgangsstellung, verschwenkt. In beiden Stellungen der Figuren 12 und 13 ist jeweils der maximale Verschwenkbereich erreicht. Korrespondierende Schnittdarstellungen zu den Figuren 12 und 13 sind die Figuren 14 und 15.

Die Figur 14 zeigt einen Schnitt durch das Zentralstück 200 in dem Bereich des Anschlagelementes 230. Das Anschlagelement 230 sieht in der Schnittdarstellung wie eine Passfeder aus, die verschieblich innerhalb des Zentralstückes 200 angeordnet ist. In der Figur 14 befindet sich ein gerundeter Endbereich 2330 mit entsprechenden Anlageflächen 233 in Anschlag mit einer korrespondierend ausgebildeten Anlagefläche 1153 in einer Ausnehmung in dem Gegenstück 115. Das Gegenstück 115 ist drehstarr an dem ersten Halter 110 verbunden. Das Gegenstück 115 befindet sich in der Ausgangsstellung, in der der erste Halter 110 entsprechend orientiert ist. In dieser Ausgangsstellung kann das Anschlagselement 230 maximal nach links parallel zur Schwenkachse 130 verschoben werden. Dadurch wird das rechte Ende des Anschlagelements 230 aus dem Freiraum innerhalb des Gegenstückes 125 des zweiten Halters 120 gebracht, sodass der zweite Halter 120 sich maximal in beide Richtungen bewegen kann. In dem dargestellten Ausführungsbeispiel wurde der Halter 120 um die Schwenkachse nach oben verschwenkt, sodass die Anlagefläche 1253 an der gerundeten Anlagefläche 233 des rechten Endes des Anschlagelements 230 anliegt. Würden sich beide Halter 110, 120 in der Ausgangsstellung befinden und das Anlagestück 230 in der Mitte befinden, würden beide Halter 110, 120 um den gleichen Winkel um die Schwenkachse 130 verschwenken können, bis sie zu einer Anlage der Anlageflächen 233, 1153, 1253 kommen würden. Je weiter das Anschlagelement 230 in die eine oder andere Richtung verschoben wird, desto mehr vergrößert oder verringert sich der mögliche Verschwenkbereich des anderen Halters in der einen oder anderen Verschwenkrichtung. Sind die Anlageflächen 1153, 1253 der Gegenstücke 115, 125 nicht gleichgeformt oder symmetrisch, können sich unterschiedliche Winkeleinstellmöglichkeiten ergeben. Neben einer gerundeten Ausgestaltung der Anlageflächen 233, 1153, 1253 können diese auch andere Formen aufweisen.

In der Figur 15 ist die umgekehrte Position gemäß Figur 13 gezeigt, das Anschlagelement 230 ist maximal nach rechts verschoben, wodurch das rechte Ende des Anschlagelementes 230 in die Ausnehmung in dem Gegenstück 125 und damit an die Anlagenflächen 1253 anstößt. Dadurch ergibt sich eine maximale Verschwenkbarkeit des ersten Halters 115 um die Schwenkachse 130.

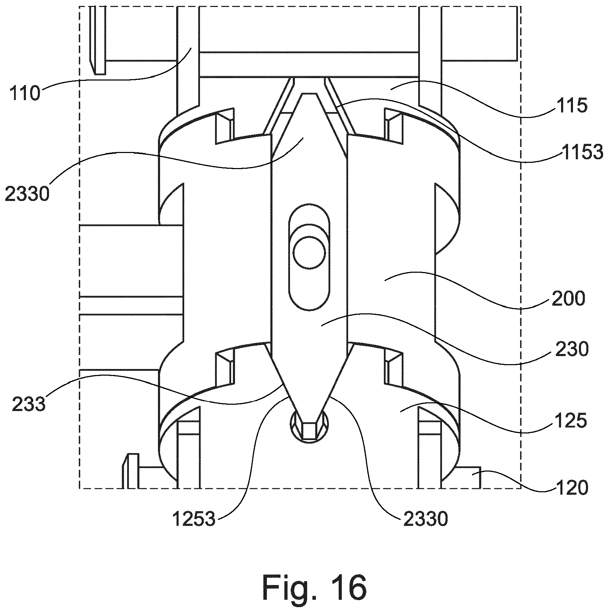

Eine alternative Ausführungsform des Anschlagelementes 230 ist in der Figur 16 gezeigt, bei der statt einer gerundeten Ausgestaltung der beiden Endstücke 2330 eine gerade, konische Ausgestaltung der Endstücke 2330 und der Anlageflächen 233 vorhanden ist. Eine korrespondierende konische Ausgestaltung der Anlageflächen 1153, 1253 ermöglicht eine große Anlagefläche und damit eine geringe Flächenpressung. Die Längsverschieblichkeit des Anschlagelementes 230 ermöglicht eine einfache Verstellung. In dem Anschlagelement 230 kann ein Langloch ausgebildet sein, durch das eine Schraube oder eine Verschiebungsbegrenzung eingeführt werden kann, um den Verstellbereich der Halter 110, 120 zueinander zu begrenzen. Das Anschlagelement 230 kann in der jeweils gewünschten Stellung fixiert werden. Die schräge Ausgestaltung der Anlageflächen 233, 1153, 1253 bedingt eine Verschiebung des Anschlagelementes 230 bei Kontakt entlang der Verschieberichtung in Richtung auf den gegenüberliegenden Halter, wodurch sich dessen Verstellwinkel in beiden Verschwenkrichtungen verändert. Der jeweilige Verschwenkbereich der Halter kann in Abhängigkeit der Stellungen der Halter zueinander innerhalb eines vorgegebenen Winkelbereiches verändert werden. Es ist vorgesehen, dass die Halter 110, 120 in der jeweils gefundenen, optimalen Stellung, in der die Befestigungselemente 10 auf der Basislage 2 aufgesetzt sind, festgehalten werden. Dies kann beispielsweise durch eine Klemmung durch die Zentralschraube 115 erfolgen.

## Patentansprüche

1. Positioniereinrichtung zum Anordnen und Ausrichten mehrerer Befestigungselemente (10) auf einer Basislage (2) eines Grundkörpers (20) einer orthopädietechnischen Einrichtung, mit einem ersten Halter (110), der zumindest eine Aufnahmeeinrichtung (111) für ein Befestigungselement (10) aufweist, und einem zweiten Halter (120), der zumindest eine Aufnahmeeinrichtung (121) für ein Befestigungselement (10) aufweist, wobei der erste Halter (110) und der zweite Halter (120) ausgehend von einer Ausgangsstellung um einen eingeschränkten Winkelbereich um eine Schwenkachse (130) verschwenkbar aneinander gelagert sind, wobei
die Halter (110, 120) an einem Zentralstück (200) schwenkbar gelagert sind und in oder an dem Zentralstück (200) eine Fixiereinrichtung (240) zur Orientierung der Positioniereinrichtung (100) auf dem Grundkörper (20) angeordnet ist.

2. Positioniereinrichtung nach Anspruch 1, wobei
jeder Halter (110, 120) mehrere Aufnahmeeinrichtungen (111, 121) für jeweils ein Befestigungselement (10) aufweist.

3. Positioniereinrichtung nach Anspruch 2, wobei
die Aufnahmeeinrichtungen (111, 121) Anlageflächen (112, 122) für das jeweilige Befestigungselement (10) aufweist, die an einem Halter (110, 120) in einer gemeinsamen Ebene liegen.

4. Positioniereinrichtung nach einem der voranstehenden Ansprüche, wobei die Aufnahmeeinrichtungen (111, 121) als Hülsen ausgebildet sind.

5. Positioniereinrichtung nach Anspruch 1, wobei
die Fixiereinrichtung (240) die Schwenkachse (130) orthogonal zu einer Gelenkachse (3) der einer an dem Grundkörper (20) anordenbaren Gelenkeinrichtung (30) ausrichtet.

6. Positioniereinrichtung nach einem der voranstehenden Ansprüche, wobei an dem Zentralstück (200) ein Anschlagelement (230) angeordnet ist, das mit einem an dem jeweiligen Halter (110, 120) angeordneten Gegenstück (115, 125) den Winkelbereich begrenzt.

7. Positioniereinrichtung nach Anspruch 6, wobei
das Anschlagelement (230) und/oder das Gegenstück (115, 125) verstellbar an dem Zentralstück (200) oder dem Halter (110, 120) angeordnet sind.

8. Positioniereinrichtung nach Anspruch 6 oder 7, wobei das Anschlagelement (230) entlang der Schwenkachse (130) verschieblich und festlegbar an dem Zentralstück (200) angeordnet ist.

9. Positioniereinrichtung nach einem der Ansprüche 6 bis 8, wobei
das Anschlagelement (230) und/oder das Gegenstück (115, 125) schräg oder gekrümmt zur Schwenkachse (130) ausgerichtete Anlageflächen (233, 1153, 1253) aufweist.

10. Positioniereinrichtung nach Anspruch 9, wobei
das Anschlagelement (230) und das jeweilige Gegenstück (115, 125) korrespondierend ausgebildete, aufeinander zu laufend orientierte, insbesondere keilförmig orientierte Anlageflächen (233, 1153, 1253) aufweist.

11. Positioniereinrichtung nach einem der Ansprüche 6 bis 10, wobei
das Anschlagelement (230) an einander entgegengesetzten Enden je einen keilförmigen Endbereich (2330) aufweist, dem jeweils ein korrespondierend geformtes Gegenstück (115, 125) zugeordnet ist.

12. Positioniereinrichtung nach einem der voranstehenden Ansprüche, wobei
die Halter (110, 120) in einem Winkelbereich von +-10° um die Ausgangsstellung verschwenkbar aneinander gelagert sind.

13. Positioniereinrichtung nach einem der voranstehenden Ansprüche, wobei
die Aufnahmeeinrichtungen (111, 121) in der Ausgangsstellung eine parallel zueinander ausgerichtete Längserstreckung aufweisen.

14. System aus Positioniereinrichtung (100) nach einem der voranstehenden Ansprüche und einem Grundkörper (20) einer Orthese oder Prothese, wobei der Grundkörper (20) ein natürliches Gelenk einer Gliedmaße übergreifend, einstückig zur Anlage an der Gliedmaße ausgebildet ist.

15. System nach Anspruch 14, wobei
an dem Grundkörper (20) eine Aufnahme (4) zur Fixierung der Positioniereinrichtung (100) angeordnet ist.

16. System nach Anspruch 15, wobei
die Aufnahme (4) in dem Bereich der Gelenkachse (3) des natürlichen Gelenkes der Gliedmaße angeordnet ist, an der die Orthese oder Prothese anordenbar ist.

17. System nach einem der Ansprüche 14 bis 16, wobei
der Grundkörper (20) an einem Modell der Gliedmaße oder der Gliedmaße selbst angeformt ist und eine der Außenkontur der Gliedmaße entsprechende Innenkontur aufweist.

18. System nach einem der Ansprüche 14 bis 17, wobei
an dem Grundkörper ein Solltrennbereich oder zumindest eine Solltrennstelle (6) ausgebildet ist, an der oder an denen der Grundkörper (20) in eine proximale und eine distale Orthesen- oder Prothesenkomponente (21, 22) teilbar ist.

## Claims

1. A positioning device for arranging and orienting a plurality of fastening elements (10) on a base layer (2) of a main body (20) of an orthopedic device, comprising a first holder (110) having at least one receiving device (111) for a fastening element (10), and a second holder (120) having at least one receiving device (121) for a fastening element (10), wherein the first holder (110) and the second holder (120) are mounted on each other so as to be pivotable about a pivot axis (130) about a limited angle range proceeding from a starting position **wherein** the holders (110, 120) are mounted pivotably on a central piece (200) and that a fixing device (240) for the orientation of the positioning device (100) on the main body (20) is arranged in or on the central piece (200).

2. The positioning device as claimed in claim 1, **wherein** each holder (110, 120) has a plurality of receiving devices (111, 121) for in each case one fastening element (10).

3. The positioning device as claimed in claim 2, **wherein** the receiving devices (111, 121) have bearing surfaces (112, 122) for the respective fastening element (10), which bearing surfaces (112, 122) lie on a holder (110, 120) in a common plane.

4. The positioning device as claimed in one of the preceding claims, **wherein** the receiving devices (111, 121) are configured as sleeves.

5. The positioning device as claimed in claim 1, **wherein** the fixing device (240) orients the pivot axis (130) orthogonally with respect to a joint axis (3) of a joint device (30) that can be arranged on the main body (20).

6. The positioning device as claimed in one of the preceding claims, **wherein** an abutment element (230) is arranged on the central piece (200) and, with a mating piece (115, 125) arranged on the respective holder (110, 120), limits the angle range.

7. The positioning device as claimed in claim 6, **wherein** the abutment element (230) and/or the mating piece (115, 125) are arranged adjustably on the central piece (200) or the holder (110, 210).

8. The positioning device as claimed in claim 6 or 7, **wherein** the abutment element (230) is arranged on the central piece (200) in such a way as to be displaceable and securable along the pivot axis (130).

9. The positioning device as claimed in one of claims 6 through 8, **wherein** the abutment element (230) and/or the mating piece (115, 125) has bearing surfaces (233, 1153, 1253) oriented obliquely or curved with respect to the pivot axis (130).

10. The positioning device as claimed in claim 9, **wherein** the abutment element (230) and the respective mating piece (115, 125) have correspondingly configured bearing surfaces (233, 1153, 1253) oriented toward each other, in particular oriented in a wedge shape.

11. The positioning device as claimed in one of claims 6 through 10, **wherein** the abutment element (230) has, at mutually opposite ends, a respective wedgeshaped end region (2330), to which a correspondingly shaped mating piece (115, 125) is assigned.

12. The positioning device as claimed in one of the preceding claims, **wherein** the holders (110, 120) are mounted on each other so as to be pivotable within an angle range of +- 10° about the starting position.

13. The positioning device as claimed in one of the preceding claims, **wherein** the receiving devices (111, 121), in the starting position, have a longitudinal extent oriented parallel to each other.

14. A system formed of a positioning device (100) as claimed in one of the preceding claims and of a main body (20) of an orthosis or prosthesis, **wherein** the main body (20), spanning a natural joint of a limb, is designed to bear integrally on the limb.

15. The system as claimed in claim 14, **wherein** a receptacle (4) for fixing the positioning device (100) is arranged on the main body (20).

16. The system as claimed in claim 15, **wherein** the receptacle (4) is arranged in the region of the joint axis (3) of the natural joint of the limb on which the orthosis or prosthesis can be arranged.

17. The system as claimed in one of claims 14 through 16, **wherein** the main body (20) is molded on a model of the limb or on the limb itself and has an inner contour corresponding to the outer contour of the limb.

18. The system as claimed in one of claims 14 through 17, **wherein** a predetermined separation region or at least one predetermined separation point (6) is formed on the main body (20), where the main body (20) can be divided into a proximal and a distal orthosis component or prosthesis component (21, 22).

## Revendications

1. Dispositif de positionnement pour agencer et aligner plusieurs éléments de fixation (10) sur une couche de base (2) d'un corps de base (20) d'un dispositif orthopédique, comprenant un premier support (110) qui comporte au moins un dispositif de réception (111) pour un élément de fixation (10), et un deuxième support (120) qui comporte au moins un dispositif de réception (121) pour un élément de fixation (10), le premier support (110) et le deuxième support (120) étant montés l'un contre l'autre de manière à pouvoir pivoter autour d'un axe de pivotement (130) à partir d'une position initiale sur une plage angulaire limitée,
dans lequel
les supports (110, 120) sont montés pivotants sur une pièce centrale (200), et un dispositif de fixation (240) est agencé dans ou sur la pièce centrale (200) pour orienter le dispositif de positionnement (100) sur le corps de base (20).

2. Dispositif de positionnement selon la revendication 1,
dans lequel chaque support (110, 120) comporte plusieurs dispositifs de réception (111, 121) pour respectivement un élément de fixation (10).

3. Dispositif de positionnement selon la revendication 2,
dans lequel les dispositifs de réception (111, 121) présentent des surfaces d'appui (112, 122) pour l'élément de fixation (10) respectif, qui se trouvent dans un plan commun sur un support (110, 120).

4. Dispositif de positionnement selon l'une des revendications précédentes, dans lequel les dispositifs de réception (111, 121) sont réalisés sous forme de douilles.

5. Dispositif de positionnement selon la revendication 1,
dans lequel le dispositif de fixation (240) vient aligner l'axe de pivotement (130) orthogonalement à un axe d'articulation (3) d'un dispositif d'articulation (30) pouvant être agencé sur le corps de base (20).

6. Dispositif de positionnement selon l'une des revendications précédentes, dans lequel un élément de butée (230) est agencé sur la pièce centrale (200), lequel délimite la plage angulaire avec une contre-pièce (115, 125) agencée sur le support respectif (110, 120).

7. Dispositif de positionnement selon la revendication 6,
dans lequel l'élément de butée (230) et/ou la contre-pièce (115, 125) sont agencés de manière réglable sur la pièce centrale (200) ou sur le support (110, 120).

8. Dispositif de positionnement selon la revendication 6 ou 7,
dans lequel l'élément de butée (230) est agencé sur la pièce centrale (200) de manière à pouvoir être déplacé et immobilisé le long de l'axe de pivotement (130).

9. Dispositif de positionnement selon l'une des revendications 6 à 8,
dans lequel l'élément de butée (230) et/ou la contre-pièce (115, 125) présente(nt) des surfaces d'appui (233, 1153, 1253) alignées en oblique ou en courbe par rapport à l'axe de pivotement (130).

10. Dispositif de positionnement selon la revendication 9,
dans lequel l'élément de butée (230) et la contre-pièce respective (115, 125) présentent des surfaces d'appui (233, 1153, 1253) réalisées de manière à se correspondre, orientées de manière à converger, en particulier orientées en forme de coin.

11. Dispositif de positionnement selon l'une des revendications 6 à 10,
dans lequel l'élément de butée (230) présente, à des extrémités opposées l'une à l'autre, une zone d'extrémité respective (2330) en forme de coin, zones à chacune desquelles est associée une contre-pièce (115, 125) de forme correspondante.

12. Dispositif de positionnement selon l'une des revendications précédentes,
dans lequel les supports (110, 120) sont montés l'un sur l'autre de manière à pouvoir pivoter dans une plage angulaire de +- 10° autour de la position initiale.

13. Dispositif de positionnement selon l'une des revendications précédentes,
dans lequel les dispositifs de réception (111, 121) présentent, dans la position initiale, des extensions longitudinales orientées parallèlement l'une à l'autre.

14. Système composé d'un dispositif de positionnement (100) selon l'une des revendications précédentes et d'un corps de base (20) d'une orthèse ou d'une prothèse,
dans lequel le corps de base (20) est réalisé d'une seule pièce pour s'appliquer sur un membre en coiffant une articulation naturelle du membre.

15. Système selon la revendication 14,
dans lequel un logement (4) pour la fixation du dispositif de positionnement (100) est disposé sur le corps de base (20).

16. Système selon la revendication 15,
dans lequel le logement (4) est disposé dans la zone de l'axe d'articulation (3) de l'articulation naturelle du membre sur lequel l'orthèse ou la prothèse peut être agencée.

17. Système selon l'une des revendications 14 à 16,
dans lequel le corps de base (20) est conformé sur un modèle du membre ou sur le membre lui-même et présente un contour intérieur correspondant au contour extérieur du membre.

18. Système selon l'une des revendications 14 à 17,
dans lequel une zone de séparation de consigne ou au moins un point de séparation de consigne (6) est formé(e) sur le corps de base, au niveau duquel ou desquels le corps de base (20) peut être divisé en composants d'orthèse ou de prothèse proximal et distal (21, 22).
